Europäisches Patentamt

European Patent Office

Office européen des brevets

(19)

(11) Veröffentlichungsnummer : **0 051 796**
**B1**

(12) **EUROPÄISCHE PATENTSCHRIFT**

(45) Veröffentlichungstag der Patentschrift :
05.10.83

(21) Anmeldenummer : 81109073.7

(22) Anmeldetag : 28.10.81

(51) Int. Cl.³ : **C 09 J   3/14**, C 08 L 33/06,
C 08 K   5/55, C 08 F   4/52,
C 08 F 20/18, A 61 K   6/08,
A 61 B 17/18

(54) Borverbindungen enthaltende lagerstabile, aerob härtende Kunststoffmassen, insbesondere Reaktionsklebstoffe.

(30) Priorität : 06.11.80 DE 3041904

(43) Veröffentlichungstag der Anmeldung :
19.05.82 Patentblatt 82/20

(45) Bekanntmachung des Hinweises auf die Patenterteilung : 05.10.83 Patentblatt 83/40

(84) Benannte Vertragsstaaten :
AT BE CH DE FR GB IT LI LU NL SE

(56) Entgegenhaltungen :
AT B 221 813
GB A 1 129 525
US A 4 182 823

(73) Patentinhaber : Henkel Kommanditgesellschaft auf
Aktien
Postfach 1100 Henkelstrasse 67
D-4000 Düsseldorf 1 (DE)

(72) Erfinder : Ritter, Wolfgang, Dr.
Oppelner Weg 3
D-4000 Düsseldorf 12 (DE)

# 0 051 796

Borverbindungen enthaltende lagerstabile, aerob härtende Kunststoffmassen, insbesondere Reaktionsklebstoffe

Die Erfindung bezieht sich auf lagerstabile Kunststoffmassen auf Basis von ethylenische Doppelbindungen enthaltenden Verbindungen, die Organo-Borverbindungen als Initiatoren enthalten und unter Einwirkung von Sauerstoff aushärten. Beschrieben werden insbesondere einkomponentige kalthärtende Reaktionsklebstoffe, die bevorzugt auf Basis von Methacrylsäurederivaten aufgebaut sind.

Klebstoffe, die durch Polymerisation von ethylenische Gruppen enthaltenden Verbindungen härten, sind seit langem bekannt. Diese lassen sich aus Methacrylsäureestern beziehungsweise den Acrylsäureestern der verschiedensten Alkohole durch Zusatz von Peroxiden beziehungsweise Hydroperoxiden und weiterer Hilfsmitteln herstellen. Darüber hinaus sind für dentalmedizinische oder chirurgische Anwendungen Binde- und Füllmittel bekannt, die neben Acrylsäure- oder Methacrylsäureestern und weiteren ethylenische Doppelbindungen enthaltenden Reaktionspartnern als wesentlichen Bestandteil Trialkylborverbindungen wie zum Beispiel Triethylbor, Tri-n-butylbor und ähnliche enthalten. Derartige Trialkylborverbindungen weisen aber den Nachteil auf, leicht entzündlich zu sein, so daß die Handhabung dieser Klebstoffe erhebliche Schwierigkeiten bereitet. Man hat diesen Übelstand dadurch zu beseitigen versucht, daß man die Trialkylborverbindungen mit 0,3 bis 0,9 Mol Sauerstoff umgesetzt hat. Außerdem hat man bereits versucht, die Trialkylborverbindungen mit Aminen umzusetzen, um so die Selbstentzündlichkeit herabzusetzen. Durch diese Maßnahmen wird die Zündtemperatur zwar in einen Bereich von 0 bis 70 °C verschoben, aber trotzdem bleibt eine erhebliche Unsicherheit bei der Handhabung derartiger Mischungen bestehen. Insbesondere eignen sie sich nicht für konstruktive Verklebungen.

Die bis heute beschriebenen Polymerisationssysteme, die Organo-Borverbindungen als reaktionsauslösende Komponente verwenden, sind sogenannte Zweikomponentensysteme, das heißt die zu polymerisierenden olefinischen Massen werden erst dann mit der getrennt gelagerten Organo-Borverbindung vermischt, wenn ihre unmittelbar anschließende Polymerisation beabsichtigt ist. Insbesondere gilt das für kalthärtende Systeme, das heißt für Reaktionsgemische, die bei Raumtemperatur oder bestenfalls mäßig erhöhten Temperaturen der Polymerisation unterworfen werden sollen. Als Literaturbeispiele für solche Organo-Borverbindungen als Starter einsetzende Reaktionssysteme sei verwiesen auf die DE-OS 23 21 215, die US-PS 4 167 616 und die GB-PS 1 113 722.

Die vorliegende Erfindung geht von der Aufgabe aus, aerob härtende Kunststoffmassen auf Basis ethylenisch ungesättigter polymerisierbarer Verbindungen unter Verwendung von Organo-Borverbindungen als Reaktionsauslöser bzw. Starter zu schaffen, die bei Ausschluß von Sauerstoff hinreichend lagerstabil sind, gleichwohl die polymerisierbaren olefinisch ungesättigten Komponenten in Mischung mit den Organo-Borverbindungen enthalten und bei Zutritt von Sauerstoff kalthärtend sind. Solche einkomponentigen Reaktionsgemische können vielseitige Anwendung, beispielsweise als Gießharze und Füllstoffe, vor allem aber auch als Klebstoffe finden. Eine besondere Aufgabe der vorliegenden Erfindung war es daher, einen Reaktionsklebstoff, das heißt einen spontan durch chemische Reaktion härtenden Klebstoff vorzuschlagen, der sich gefahrlos handhaben läßt und bei praktikablen Standzeiten zu guten Verklebungen führt, auch an solchen Materialien, die sich nur schlecht trocknen lassen.

Gegenstand der Erfindung sind dementsprechend in einer ersten Ausführungsform unter Sauerstoffausschluß lagerstabile, fließ- bzw. streichfähige Kunststoffmassen auf Basis polymerisierbarer olefinisch ungesättigter Verbindungen und Organo-Borverbindungen als Starter, die dadurch gekennzeichnet sind, daß sie als aerob härtende Einkomponentengemische vorliegen und im wesentlichen bestehen aus

a) für die Auslösung einer Polymerisation ethylenisch ungesättigter Verbindungen geeignete Organo-Borverbindungen,

b) wenigstens eine ethylenische Bindung enthaltende Verbindungen mit einem Molekulargewicht zwischen 63 und 10 000,

c) Inhibitoren bzw. Stabilisatoren gegenüber anionischer Polymerisation sowie

d) weitere Hilfsstoffe wie Stabilisatoren gegen radikalische Polymerisation, Verdickungsmittel, Hilfsmonomere, Farbstoffe und Pigmente.

In weiteren Ausführungsformen betrifft die Erfindung das Verfahren zur Herstellung solcher lagerstabilen, fließ- bzw. streichfähigen Kunststoffmassen sowie ihre Verwendung, insbesondere als Reaktionsklebstoff zum Verbinden von Metall, Holz, Glas, Keramik und Kunststoffen sowie auf chirurgischem und dentalmedizinischem Gebiet.

In einer besonders wichtigen Ausführungsform bezieht sich die Erfindung demnach auf einkomponentige, aerob-härtende, hochfeste Konstruktionsklebstoffe, die charakterisiert sind im wesentlichen durch den Gehalt an wenigstens einem polymerisierbaren Monomeren, einem oder mehreren Boralkylen, geeigneten Stabilisatoren gegen vorzeitige Polymerisation, einem oder mehreren Polymeren zur Erhöhung der Kohäsion und zur Einstellung der gewünschten Viskosität.

Die Klebstoffmischung wird unter vollständigem Sauerstoffausschluß hergestellt. Man arbeitet zweckmäßig unter einer Schutzgasatmosphäre aus reinem Stickstoff oder Edelgasen wie Argon und dergleichen. Die Härtung des Klebstoffes tritt ein, sobald die Mischung in Kontakt mit Luft tritt. Die offene Zeit kann innerhalb weiter Grenzen variiert werden.

2

Die erfindungsgemäß als Starter dienenden Organo-Borverbindungen können als solche den reaktiven Massen zugesetzt werden, sie können sich aber — und hier liegt eine bevorzugte Ausführungsform der Erfindung — wenigstens anteilsweise auch in situ aus Borhydridverbindungen und olefinisch ungesättigten Komponenten der reaktiven Massen bilden. Die als Starter geeigneten Organo-Borverbindungen enthalten wenigstens eine B-C-Bindung, vorzugsweise wenigstens 2 solcher B-C-Bindungen. Im allgemeinen sind in den erfindungsgemäß zum Einsatz kommenden reaktiven Massen alle drei Valenzen des Bors an Kohlenstoff bzw. Kohlenwasserstoffreste gebunden. In Betracht kommen dabei sowohl Boralkylverbindungen als auch Borarylverbindungen. Bevorzugt ist wenigstens eine Valenz des Bors an ein aliphatisches Kohlenstoffatom gebunden. Eine besonders bevorzugte Klasse der Organo-Borverbindungen sind dementsprechend Boralkyle mit gleichen oder unterschiedlichen Alkylresten. Die Alkylreste können gerad- oder verzweigtkettig oder auch Cycloalkylreste sein, wobei diese Reste auch ihrerseits zu einem oder mehreren Ringsystemen zusammengeschlossen sein können. Jeder Alkylrest kann beispielsweise bis zu 25 C-Atome aufweisen. Bevorzugt sind die Alkylreste gesättigt. Neben oder anstelle der Alkylreste können aber auch Arylreste an das Bor gebunden sein. Bevorzugt sind hier einkernige Arylreste, höhere Reste, insbesondere zweikernige Arylreste, sind jedoch nicht ausgeschlossen.

Werden die als Starter dienenden Organo-Borverbindungen nicht als solche in den Klebstoffmassen eingesetzt, sondern in der Masse oder in einem Anteil der Reaktionsmasse in situ gebildet, so ist zu unterscheiden zwischen den beim Herstellungsverfahren einzusetzenden Borverbindungen und den letztlich in den reaktiven Einkomponentenmassen vorliegenden Organo-Borverbindungen. In dieser Ausführungsform macht sich die Erfindung die Tatsache zu nutze, daß Borhydridbindungen im allgemeinen problemlos mit olefinischen Doppelbindungen unter Addition der Borhydridvalenz an die Kohlenstoffdoppelbindung reagieren. So können also im Rahmen der Herstellung der reaktiven Einkomponenten-Gemische zur in situ-Bildung des Starters Borhydridverbindungen zum Einsatz kommen. Geeignet sind dabei sowohl Diboran als auch Organo-Borhydride mit einem oder mit zwei Kohlenwasserstoffresten am Bor.

Die vorliegenden B-H-Bindungen reagieren üblicherweise bei Raumtemperatur mit sterisch nicht gehinderten olefinischen Doppelbindungen unter Ausbildung zusätzlicher B-C-Bindungen.

Wegen der bekannten leichten Entflammbarkeit niedriger Boralkyle kann es aus praktischen Gründen zweckmäßig sein, aus der großen Klasse der an sich bekannten Organo-Borverbindungen solche Komponenten auszuwählen, die eine verringerte Entflammbarkeit besitzen. Hierdurch wird die Herstellung der Reaktionsmassen erleichtert. Es hat sich allerdings gezeigt, daß in den fertigen Reaktionsmassen der Erfindung die in nur beschränkten Mengen homogen verteilt vorliegenden Organo-Borverbindungen auch dann unbedenklich sind, wenn diese Starterverbindungen als solche leicht entflammbar sind.

Als Borverbindungen, die als Initiator wirken, kommen demnach zahlreiche bekannte beziehungsweise auf bekannte Weise herstellbare Boralkyle in Frage. Typische Vertreter dieser Borverbindungen sind beispielsweise 9-Borabicyclo [3.3.1] nonan, Diisopinocampheylboran, Dicyclohexylboran, Thexylboran-(2,3-dimethyl-2-butyl-boran), 3,5-Dimethylborinan, Diisoamylboran. Unter diesen Verbindungen ist das zuerst genannte 9-Bora-bicyclo [3.3.1] nonan aus praktischen Gründen bevorzugt. Die vorstehend genannten Verbindungen können beispielsweise aus Natriumborhydrid und Bortrifluorid mit geeigneten Olefinen oder Diolefinen hergestellt werden. Auch können zur Darstellung Diboran, dessen Ether-, Amin- oder Sulfidkomplexe eingesetzt werden.

Eine Zusammenstellung der Herstellungsmöglichkeiten geeigneter Borverbindungen findet sich in der Monographie Herbert C. Brown, 1975 « Organic Synthesis via Boranes », Verlag John Wiley & Sons. Als Initiatoren können weiterhin einfache Trialkylborane sowie Hydroborierungsprodukte aus B-H-Verbindungen wie Monoalkyl- und Dialkylboranen und Olefinen eingesetzt werden. Als Olefine sind z. B. brauchbar Ethen, Propen, Buten, Isobuten, Hexen, Cyclohexen, Vinylchlorid, Allylchlorid, Allylamin oder auch Methacrylsäuremethylester, Vinylacetat oder Crotonsäure-methylester. Unter den geeigneten Verbindungen sind beispielsweise erwähnenswert :

Trimethylbor, Triethylbor, Tripropylbor, isomeres Tributylbor, isomeres Trihexylbor, Diisopinocampheylbutylbor, Thexylcyclohexylcyclopentylbor, Thexyllimonylbor, Trinorbornylbor, B-Butyl-9-borabicyclo[3.3.1]bor, B-Isobutyl-9-borabicyclo[3.3.1]bor, B-[2-(4-Cyclohexenyl)-ethyl]-9-borabicyclo[3.3.1]bor, B-Cyclopropyl-9-borabicyclo[3.3.1]nonan, B-p-Tolyl-9-borabicyclo[3.3.1]nonan, B-tert.-Butyl-3,5-dimethylborinan. Weiter sind typische Vertreter für geeignete Borverbindungen das Umsetzungsprodukt von 1,2-Dihydroxybenzol mit Borwasserstoff (Catecholboran) und Tri-n-butylboroxin. Auch alkylierte $(C_{1-9})$ Dihydroxybenzole sind als Ausgangsmaterial brauchbar.

Von den einzusetzenden Initiatoren verwendet man üblicherweise bis zu 15 Gewichtsprozent, insbesondere 0,01 bis 15 Gewichtsprozent, bezogen auf den polymerisierbaren Anteil. Mengen von wenigstens etwa 0,1 Gewichtsprozent der Organo-Borverbindungen können besonders bevorzugt sein, wobei hier wiederum der Bereich von etwa 0,1 bis 10 Gewichtsprozent besondere Bedeutung hat. 0,5 bis 10 % und insbesondere 1 bis 5 Gewichtsprozent sind bei der Wahl geeigneter reaktiver Borverbindungen die besonders wichtigen Bereiche.

Dabei kann die Gesamtmenge der Starterverbindung der Reaktionsmasse von Anfang an zugesetzt werden — bzw. in situ in ihr gebildet werden —, in einer bevorzugten im folgenden noch zu schildernden Ausführungsform wird jedoch nur ein Anteil der Starterverbindung bei der Herstellung der reaktiven Massen vorgelegt, während der noch benötigte zweite Anteil in einem abschließenden Verfahrensschritt der Reaktionsmasse zugesetzt bzw. in situ in ihr gebildet wird.

Als polymerisierbarer Bestandteil der erfindungsgemäßen reaktiven Kunststoffmassen kommen zahlreiche Verbindungen in Frage, die ethylenische Doppelbindungen enthalten. Für das wichtige Gebiet der Einkomponenten-Reaktionsklebstoffe haben besondere Bedeutung Derivate der Methacrylsäure und/oder der Acrylsäure, im folgenden als (Meth)acrylsäurederivate bezeichnet. Besonders wichtig sind hier die Ester und/oder Säureamide dieser Komponenten, wobei die Säureamide ihrerseits auch im Amidstickstoff substituiert sein können. In Betracht kommen zum Beispiel die (Meth)acrylsäureester von monovalenten Alkoholen, also etwa (Meth)acrylsäuremethylester, (Meth)acrylsäureethylester, (Meth)acrylsäurebutylester und (Meth)acrylsäureethylhexylester, die (Meth)acrylsäureester von polyvalenten Alkoholen, wie zum Beispiel Ethylenglycol, Diethylenglycol, Polyethylenglycol und Trimethylolpropan, die Di- und Mono(meth)acrylsäureester von Glycerin, die Di(meth)acrylsäureester von Tri- und Tetraethylenglycol, von Di-, Tri-, Tetra- und Pentapropylenglycol, die Di(meth)acrylsäureester von ethoxyliertem oder propoxyliertem Diphenylolpropan. Auch kommen (Meth)acrylsäureester von Alkoholen in Frage, die sich vom Dihydroxymethyltricyclodecan ableiten oder auch solche, die auf Basis von Tricyclodecan hergestellt worden sind, wobei 2 alkoholische Funktionen im Ringsystem durch Umsetzung mit Dicarbonsäuren wie Maleinsäure oder auch Cyclohexandicarbonsäure oder Terephthalsäure verlängert sind. Des weiteren sind einsetzbar Umsetzungsprodukte des Diglycidylethers von Diphenylpropan mit Methacrylsäure und/oder Acrylsäure. Auch sind Reaktionsprodukte aus Diisocyanaten oder Triisocyanaten zum Beispiel Toluylendiisocyanat, Diphenylmethandiisocyanat, Isophorondiisocyanat, trimerisiertem Toluylendiisocyanat und dergleichen mit Hydroxyalkyl(meth)acrylaten als polymerisierbare Bestandteile brauchbar.

Prinzipiell brauchbar sind erfindungsgemäß aber auch polymerisierbare Monomere wie Vinylacetat, Vinylhalogenide wie Vinylchlorid, Vinylfluorid, Vinylbromid, Styrol, Divinylbenzol, Crotonsäure- und Maleinsäureester oder die sogenannten gegebenenfalls styrolisierten ungesättigten Polyesterharze. In Reaktionsklebstoffen werden diese Verbindungen im allgemeinen nur in untergeordneter Menge mitverwendet.

Außerdem kommen in Frage 2-Acryloyloxyethylphosphat, 2-Methacryloyloxyethylphosphat, Bis-2-acryloyloxyethylphosphat, Bis-2-methacryloyloxyethylphosphat, Tris-2-acryloyloxyethylphosphat, Tris-2-methacryloyloxyethylphosphat und Säureamide wie etwa Dimethylenbis(meth)acrylamid, Tetramethylenbis(meth)acrylamid, Trimethylhexamethylenbis(meth)acrylamid, Tri(meth)acryloyldiethylentriamin und dergleichen mehr.

Ganz allgemein liegt das Schwergewicht bei den zu polymerisierenden Komponenten auf Verbindungen mit einer oder mit zwei olefinischen Doppelbindungen im Molekül. Die Mitverwendung von höher ungesättigten Komponenten ist zwar nicht ausgeschlossen, es muß jedoch berücksichtigt werden, daß ihre Gegenwart durch Vernetzung zu einer Versprödung der ausgehärteten Massen führen kann.

Ein weiteres wesentliches Element der Erfindung liegt in der Mitverwendung von Inhibitoren bzw. Stabilisatoren gegenüber anionischer Polymerisation. Im Rahmen der Erfindung wurde festgestellt, daß Organo-Borverbindungen, insbesondere Boralkyle, auch bei vollständiger Abwesenheit von Sauerstoffspuren eine polymerisationsauslösende Wirkung zeigen. Dieser Polymerisationsvorgang läuft allerdings sehr viel langsamer ab als die durch Sauerstoff initiierte radikalische Reaktionsauslösung. Offensichtlich handelt es sich bei diesem Reaktionsmechanismus um eine milde anionische Polymerisationsauslösung, die durch Temperaturerhöhung aktiviert werden kann. Wie noch geschildert wird, macht sich die Erfindung gezielt von dieser doppelten Möglichkeit der Reaktionsauslösung durch Organo-Borverbindungen Gebrauch.

Für die Lagerbeständigkeit der erfindungsgemäßen reaktiven Kunststoffmassen ist es damit allerdings von Bedeutung, den anionischen Polymerisationseffekt und die mit ihm verbundene Eindickung der Reaktionsmasse zum gezielten Zeitpunkt abzustoppen bzw. zu unterbinden. Hierzu dient die erfindungsgemäß vorgesehene Mitverwendung von Inhibitoren bzw. Stabilisatoren gegenüber einer anionischen Polymerisation.

Als solche Inhibitoren bzw. Stabilisatoren sind insbesondere die Verbindungen gut geeignet, die auch zum Stabilisieren von $\alpha$-Cyanacrylsäureestern geeignet sind, zum Beispiel saure gasförmige Stoffe wie $SO_2$, $NO_2$, NO, $CO_2$, HCl und HF. Weiter seien in diesem Zusammenhang als mögliche Inhibitoren beziehungsweise Hilfsinhibitoren gegen vorzeitige Härtung des Gemischs erwähnt Phosphorsäure, Borsäure, Borsäureester, Sulfonsäuren oder Sultone, Carbonsäuren wie (Meth)acrylsäure, Essigsäure, Trichlor- und Trifluoressigsäure, Carbonsäureanhydride oder Alkohole wie zum Beispiel Hydroxyethylmethacrylat, Phosphorpentoxid.

In vielen Fällen kann es zweckmäßig sein, als Inhibitoren gegen die anionische Polymerisation Komponenten einzusetzen, die einer radikalischen Polymerisation gegenüber reaktiv sind und damit in die aushärtende Reaktionsmasse eingebunden werden. Geeignet sind hier insbesondere freie Carbonsäuren mit olefinischen Doppelbindungen, insbesondere also Acrylsäure und/oder Methacrylsäure. Ihr Zusatz in Einkomponenten-Reaktionsklebstoffen gemäß der Erfindung hat dementsprechend mehrfache Bedeutung. Bekanntlich führen freie Carboxylgruppen in der Klebstoffmasse zur Verbesserung deren Adhäsionsfähigkeit, gleichzeitig stabilisieren die freien Säuren die reaktiven Einkomponentengemische gegen eine unerwünschte anionische Polymerisation.

Auch können im Gemisch als Komplexierungsmittel wirksame Substanzen wie Amine und Ester, zum Beispiel Pyridin, Propylamin, Butylamin, Allylamin, Diisopropylamin, N,N-Dimethyltoluidin, Benzoesäuremethylester oder -ethylester oder Anissäuremethylester oder -ethylester günstigen Einfluß auf die Stabilität haben.

Im allgemeinen ist dafür zu sorgen, daß auch die vorzeitige radikalische Polymerisation verhindert wird. Hier bieten sich zunächst Hydride wie Natriumborhydrid, Lithiumaluminiumhydrid und Calciumhydrid an. Weiter können für diesen Zweck Cumol, Hydrochinon, 2,6-Di-tert.-butyl-p-cresol, 2,6-Di-tert.-butyl-4-methoxyphenol, Bis(2-hydroxy-3-tert.-butyl-5-methyl-phenyl)methan, Bis(3,5-di-tert.-butyl-4-hydroxyphenyl)methan, Galvinoxyl, Bis(2-hydroxy-3-tert.-butyl-5-methyl-phenyl)sulfid, Bis(3-tert.-butyl-4-hydroxy-5-methylphenyl)sulfid oder auch Diphenylamin, N,N'-Di-phenyl-p-phenylendiamin, Phenothiazin, 2-Phenylbenzimidazol, Anilin, Dinitrobenzol, 2-Nitro-α-naphthol, Tetraphenylethylen und Triphenylmethan brauchbar sein.

Zur wirkungsvollen Verhinderung einer radikalischen Polymerisation sind besonders sehr stark wirksame Antioxidantien geeignet. Beispiele sind Galvinoxyl, Kupfer-II-dibutyldithiocarbamat, Phenothiazin, und Zinkdiethyldithiocarbamat. In einer weiteren bevorzugten Ausführungsform der Erfindung erfolgt jedoch diese abschließende Stabilisierung gegen radikalische Polymerisation durch praktisch quantitative Beseitigung unausweichlich eingeschleppter Sauerstoffspuren mittels Borhydridverbindungen, insbesondere durch Zusatz von Organo-Borhydriden wie Dialkylbormonohydriden. Die Borhydridbindung vernichtet aufgrund ihrer hohen Reduktionsbereitschaft vorliegende Sauerstoffspuren praktisch quantitativ. Die Auswahl der zudosierten Menge an Organo-Borhydridverbindungen ist dabei unproblematisch. Sie kann im Überschuß erfolgen. Der überschießende Betrag an Organo-Borhydrid setzt sich mit den olefinischen Komponenten des Reaktionsgemisches zur Boralkylverbindung um, die ihrerseits dann wieder die Funktion des Starters übernimmt.

Die erfindungsgemäßen Kunststoffmassen sollen zur erleichterten Applikation in der Regel fließfähig sein oder streichfähige Konsistenz besitzen. Besonders geeignet ist der Viskositätsbereich von 200 bis 100 000 mPas wobei Werte im Bereich von etwa 1 000 bis 20 000 mPas besondere Bedeutung haben können. Brauchbare Klebstoffe liegen beispielsweise bei Viskositätswerten im Bereich von einigen Tausend mPas. Die flüssige bzw. streichfähige Konsistenz der Massen beruht in der Regel auf der Beschaffenheit der polymerisierbaren Monomerkomponenten. Häufig handelt es sich hier um bei Normaltemperatur leicht bewegliche Flüssigkeiten, die eingedickt werden müssen, um beispielsweise Klebstoffkonsistenz einzustellen. Diese Eindickung erfolgt durch Mitverwendung von vorzugsweise homogen mischbaren Polymerkomponenten wie Polymerisate von (Meth)acrylsäurederivaten, entsprechenden Copolymeren, Polyvinylacetat, chlorsulfoniertem Polypropylen, Polychloropren, Polyurethanen oder dergleichen. Schwierig ist es allerdings, getrennt hergestellte Polymerisate derart von Restsauerstoffspuren zu befreien, daß nicht unerwünschte Sekundärreaktionen im Stoffgemisch ausgelöst werden. Es hat sich deshalb als besonders vorteilhaft herausgestellt, diesen verdickenden Polymeranteil in situ durch Teilpolymerisation der gewählten Monomeren bzw. monomeren Mischung herzustellen. Hierzu kann die vergleichsweise langsam wirkende anionische Reaktionsauslösung durch Organo-Borverbindungen, insbesondere Boralkyle, eingesetzt werden. Hierzu wird also die gewählte Monomermischung durch Boralkyle unter vollkommenem Sauerstoffausschluß — zunächst ohne Zusatz von Inhibitoren — soweit vorpolymerisiert, bis die gewünschte — beispielsweise für Verklebungszwecke geeignete — Viskosität erreicht ist. Anschließend wird dann der Inhibitor gegen anionische Polymerisation zugegeben, wodurch die weitere Eindickung des reaktiven Gemisches unterbunden wird. Solche Mischungen sind bei sicherem Sauerstoff — bzw. Luftausschluß über längere Zeiträume lagerstabil, können aber gleichwohl jederzeit bei Luftzutritt zum reaktiven Einsatz kommen. In ähnlicher Weise ist es allerdings auch möglich, die hochgereinigte Monomermischung, die weder Initiatoren noch Inhibitoren enthält, thermisch vorpolymerisieren zu lassen und dann Initiatoren und Inhibitoren zuzugeben.

In vielen Fällen ist es zweckmäßig, weitere Hilfsstoffe wie Füllstoffe, zum Beispiel Quarzmehl oder dergleichen zuzugeben. Schließlich kann es zweckmäßig sein, mit geeigneten Farbstoffen bzw. Pigmenten zu färben. Wichtig ist, daß alle Zusatzstoffe wirklich frei oder wenigstens möglichst weitgehend frei von Sauerstoff sind.

Da beim praktischen Arbeiten ein totaler Luft- bzw. Sauerstoffausschluß in der Regel nicht zu verwirklichen ist, dessen Abwesenheit für die Lagerstabilität der Einkomponentengemische aber eine Voraussetzung ist, ist die abschließende Stabilisierung gegen radikalische Polymerisationen vorgesehen. Wie bereits erwähnt, können hierzu hochwirksame Antioxidationsmittel Verwendung finden. Hier sollte dann allerdings darauf geachtet werden, daß keine wesentlichen Überschüsse dieser stabilisierenden Komponenten vorliegen, anderenfalls könnten Beeinträchtigungen bei der Anwendung der Kunststoffmassen und ihrer hier gewünschten oxidativen Härtung auftreten. Der bereits erwähnte bevorzugte Weg der erfindungsgemäßen abschließenden Stabilisierung ist die Zugabe von Borhydridverbindungen, die zunächst unvermeidlich eingeschleppte Sauerstoffspuren reduzieren und damit deaktivieren, während derÜberschuß der Borhydridkomponenten zusätzliche Starterverbindungen bildet. Bei der Durchführung dieser Ausführungsform des erfindungsgemäßen Herstellungsverfahrens kann es also zweckmäßig sein, einen substantiellen Anteil der borhaltigen Starterverbindungen — beispielsweise ein Viertel bis zwei Drittel dieser Komponente — erst im letzten Verfahrensschritt der abschließenden Stabilisierung gegen

0 051 796

Radikalpolymerisation dem Reaktionsgemisch zuzusetzen.

Besonders interessante Ergebnisse liefern die erfindungsgemäßen neuen Reaktionsklebstoffe auf dem Gebiet der medizinischen Anwendung, insbesondere bei der Verklebung von Knochen sowie auf dem Dentalgebiet. Trotz der hier gegebenen Schwierigkeiten die miteinander zu verklebenden Elemente an den mit Klebstoff zu bestreichenden Flächen vollständig zu trocknen und/oder zu reinigen, lassen sich Verklebungen erstaunlich hoher Festigkeit erreichen.

Die neuen Klebstoffe zeichnen sich dadurch aus, daß sie bei Raumtemperatur eine hohe Härtungsgeschwindigkeit haben und bereits nach kurzer Zeit gute Festigkeiten auf einer Vielzahl von unterschiedlichen Oberflächen aufweisen. Hervorzuheben ist insbesondere, daß auch auf nicht völlig trockenen Oberflächen eine schnelle und gute Haftung erzielt wird. Die Klebstoffe können demnach eingesetzt werden als sogenannte Konstruktionsklebstoffe zur Verklebung von Metallen, Holz, Glas Keramik und Kunststoffen. Gegenüber anderen reaktiven Flüssigklebstoffen wie den Cyanacrylaten weisen die erfindungsgemäßen Systeme den Vorteil fast völliger Unabhängigkeit in der Auswahl des Monomeren beziehungsweise der Monomerenmischung auf. Sie haben damit einen äußerst breiten Spielraum beim Einsatz von Monomermischungen. Gute Kohäsion und hohe Adhäsion auf unterschiedlichen Oberflächen sind gewährleistet. So erfolgt auf nahezu allen Oberflächen, unabhängig vom Metall, durch Luftsauerstoff, der in praktisch konstanter Konzentration überall vorliegt, die Aushärtung.

Zur Herstellung der einkomponentigen reaktiven Kunststoffmischungen wird die benötigte Menge des getrockneten Monomeren bzw. monomeren Gemisches zweckmäßigerweise zunächst in einer Hochvakuumapparatur entgast, gegebenenfalls destilliert, und gewünschtenfalls anschließend in ein Vorratsgefäß umkondensiert. Gewünschtenfalls kann zur weiteren Reinigung mit hydrierenden Substanzen, zum Beispiel $NaBH_4$, $LiAlH_4$, $CaH_2$ oder aber auch mit einer Organo-Borhydridverbindung vorbehandelt werden, wie es zuvor für den abschließenden Reinigungsschritt zur Beseitigung von Sauerstoffspuren geschildert worden ist. In die sauerstofffreie Monomermischung wird dann wenigstens ein Teil der borhaltigen Starterkomponenten bzw. der Borhydridverbindungen, die mit dem Monomerengemisch borhaltigen Starter bilden, eingetragen. Soweit erforderlich wird durch gesteuerte anionische Polymerisation unter dem Einfluß der Organo-Borverbindungen die Viskosität des Gemisches in den gewünschten Bereich angehoben. Anschließend werden die Inhibitoren gegen die ionische Polymerisation zugesetzt und damit diese Verfahrensstufe abgebrochen. Gewünschtenfalls werden weitere Zusatzstoffe wie Füllmittel, Pigmente und dergleichen dem Gemisch zugegeben. Abschließend wird die Stabilisierung gegen radikalische Polymerisation durch Beseitigung der vorliegenden Sauerstoffspuren durch Zugabe von Borhydridverbindungen und/oder durch Zugabe von Antioxidationsmitteln durchgeführt.

Für die Herstellung von Reaktionsklebstoffen der erfindungsgemäßen Art können beispielsweise der sauerstofffreien Monomermischung unter reinem Stickstoff die folgenden Mengen an Reaktanten bzw. potentiellen Reaktanten zugegeben werden.

0,01 bis 15 Gew.% Borverbindung
0,01 bis 10 Gew.%, insbesondere 0,01 bis 5 Gew.% Inhibitoren gegen ionische Polymerisation
0     bis 40 Gew.% eines Polymeren
0,01 bis  3 Gew.% an Radikalinhibitoren.

Die reaktiven Kunststoffgemische sind unter Ausschluß von Luftsauerstoff aufzubewahren. Der auf ausgetragene Anteile der Reaktionsmasse einwirkende Luftsauerstoff löst die Polymerisation aus und führt zur Härtung.


Beispiel 1


Unter Sauerstoffausschluß werden 12,2 g 9-Borabicyclo[3.3.1]nonan (= 9-BBN im nachfolgenden Text) in 100 ml wasserfreiem, entgastem Tetrahydrofuran gelöst. Zur Lösung werden unter Sauerstoffausschluß 8,6 g entgastes Methacrylsäuremethylester zugetropft. Das Tetrahydrofuran wird nach Beendigung der exothermen Reaktion im Vakuum abgezogen.

In einer Glovebox werden 4 g Polymethacrylsäuremethylester, der unter Sauerstoffausschluß mit Azoisobuttersäurenitril als Radikalstarter dargestellt worden ist, in 4,5 g sauerstofffreiem Methacrylsäuremethylester und 0,5 g sauerstofffreier Methacrylsäure gelöst. Zur Mischung werden 20 mg $NaBH_4$, 0,35 g sauerstofffreies Dimethyltoluidin und 0,5 g des Reaktionsproduktes von 9-BBN mit Methacrylsäuremethylester zugegeben. Das Gefäß wird verschlossen und unter Stickstoff in der Glovebox aufbewahrt.

Mit dieser Mischung werden nach 10 Stunden an der Luft nach DIN 68602 Buchenhölzer verklebt und nach 24 Stunden zerrissen. Die durchschnittliche Zugscherfestigkeit beträgt 8,3 N/mm².

Nach DIN 53281/53282 werden sandgestrahlte und entfettete Eisenbleche verklebt und nach 24 Stunden zerrissen. Die durchschnittliche Zugscherfestigkeit beträgt 14,3 N/mm².

Bei den folgenden Klebstoffmischungen wird analog vorgegangen. In der nachfolgenden Tabelle I sind in Abhängigkeit von der laufenden Nummer des Beispiels angeführt der Bor enthaltende Starter (B-Starter), Zusätze und Monomere sowie etwaige polymere Verdingungsmittel. Es bedeutet

6

MMA = Methacrylsäuremethylester.

Tabelle I

| Beispiel Nr. | B-Starter | Zusätze | Monomer |
|---|---|---|---|
| 2 | 0,3 g 9-BBN | 20 mg NaBH$_4$ 0,35 g Benzoe-säuremethyl-ester | 10 ml MMA |
| 3 | 0,3 g 9-BBN | 20 mg NaBH$_4$ 0,4 g Anissäure-methylester | 10 ml MMA |
| 4 | 0,3 g 9-BBN | 20 mg NaBH$_4$ 0,35 g Dimethyl-toluidin | 10 ml MMA |
| 5 | 0,3 g 9-BBN | 20 mg Hydrochinon 0,4 g Anissäure-ethylester | 7 g MMA |
| 6 | 0,3 g 9-BBN | 20 mg Hydrochinon 0,35 g Dimethyl-toluidin | 7 g MMA |
| 7 | 0,3 g Tri-n-butyl-boroxin | 20 mg Hydrochinon | 7 g MMA |

In der nachfolgenden Tabelle II sind durchschnittliche Zugscherfestigkeitswerte nach DIN wiedergegeben und zwar folgen in Abhängigkeit von der laufenden Nummer des Beispiels der Zeitpunkt in Stunden, nachdem nach der Herstellung der Mischung die Verklebung durchgeführt wurde. Nach 24 Stunden wurden die Eisenbleche beziehungsweise Buchenholzprüfkörper zerrissen.

Tabelle II

| Beispiel | Verklebung in Std. nach Herstellung der Mischung | Zugscherfestigkeit bei | |
|---|---|---|---|
| | | Eisen $N/mm^2$ | Holz $N/mm^2$ |
| 2 | 200 | 23,7 | 6,0 |
| 3 | 200 | 30,2 | 8,4 |
| 4 | 150 | 26,0 | |
| 5 | 150 | 15,7 | |
| 6 | 150 | 18,6 | |
| 7 | 75 | 11,0 | |

Beispiel 8

300 ml Methylmethacrylat werden mit Natriumsulfat getrocknet, in einer Hochvakuumsapparatur bei $10^{-3}$ Torr entgast und auf 2,2 g 9-BBN kondensiert. Nach einer Stunde wird erneut entgast und auf 9 g

7

9-BBN umkondensiert. Diese Lösung wird 24 Std. gelagert, bis eine erhöhte Viskosität auftritt, die sich gut für Metallverklebungen eignet (Stammlösung in Beispiel 9 beziehungsweise 10).

Zu 25 ml dieser Lösung werden unter vollkommenem Sauerstoffausschluß 1,0 g absolut sauerstofffreie Methacrylsäure und anschließend 0,75 g 9-BBN zugegeben. Diese Mischung wird 30 Tage gelagert. Die Viskosität verändert sich im Verlaufe der Lagerung nicht. Mit dieser Mischung werden nach DIN 68602 Eisen-beziehungsweise Aluminiumblech verklebt und nach 12 Std. Zugscherfestigkeiten von 26 beziehungsweise $18 N/mm^2$ erhalten.

### Beispiel 9

Zu 25 ml der Stammlösung aus Beispiel 8 werden unter absolutem Sauerstoffausschluß 0,24 g Pyridin, 2,2 g Methacrylsäure und 0,75 g 9-BBN zugegeben. Nach 30 Tagen Lagerung werden mit diesem Klebstoff nach DIN 68602 folgende Zugscherfestigkeiten erzielt :

Eisen : 20 $N/mm^2$
Aluminium : 8 $N/mm^2$
Die Prüfkörper wurden 12 Std. nach dem Verkleben zerrissen.

### Beispiel 10

Zu 25 ml der Stammlösung aus Beispiel 8 werden unter absolutem Sauerstoffausschluß 0,24 g Pyridin und 0,75 g 9-BBN-zugegeben. Nach 30 Tagen Lagerung des Klebstoffs werden nach DIN 68602 folgende Zugscherfestigkeiten erzielt :

Eisen : 15 $N/mm^2$
Aluminium : 9 $N/mm^2$
Die Prüfkörper wurden 12 Std. nach dem Verkleben zerrissen.

### Beispiel 11

Zu 25 ml der Stammlösung aus Beispiel 8 werden unter absolutem Sauerstoffausschluß 100 mg Galvinoxyl zugegeben. Nach 30 Tagen Lagerung des Klebstoffes werden nach DIN 68602 folgende Zugscherfestigkeiten erzielt :

Eisen : 19 $N/mm^2$
Aluminium : 15 $N/mm^2$
Die Prüfkörper wurden 12 Stunden nach dem Verkleben zerrissen.

### Beispiel 12

170 ml Methylmethacrylat werden mit Natriumsulfat getrocknet, in einer Hochvakuumapparatur bei $10^{-3}$ Torr entgast und auf 2,2 g 9-BBN umkondensiert. Zu dieser Lösung werden unter absolutem Sauerstoffausschluß 15 g sauerstofffreies, umkondensiertes Hydroxyethylmethacrylat zugegeben. Diese Mischung wird 72 Std. gelagert, bis eine erhöhte Viskosität auftritt, die sich gut für Metallverklebungen eignet.

Zu 25 ml dieser Lösung werden in der Glovebox unter absolutem Sauerstoffausschluß 0,48 g Pyridin und 0,75 g 9-BBN zugegeben. Die Mischung wird 30 Tage gelagert. Die Viskosität ändert sich im Verlaufe der Lagerung nicht. Mit dieser Mischung werden nach DIN 68602 Eisen- beziehungsweise Al-Bleche verklebt und folgende Festigkeiten erzielt :

Eisen : 28 $N/mm^2$
Aluminium : 19 $N/mm^2$
Die Prüfkörper wurden 12 Std. nach dem Verkleben zerrissen.

**Ansprüche**

1. Unter Sauerstoffausschluß lagerstabile, fließ- bzw. streichfähige Kunststoffmassen (Klebstoffe, Gießharze, Füllstoffe) auf Basis polymerisierbarer olefinisch ungesättigter Verbindungen und Organo-Borverbindungen als Starter, dadurch gekennzeichnet, daß sie als aerob härtende Einkomponentengemische vorliegen, im wesentlichen bestehend aus

a) für die Auslösung einer Polymerisation ethylenisch ungesättigter Verbindungen geeignete Organo-Borverbindungen,

b) wenigstens eine ethylenische Bindung enthaltende Verbindungen mit einem Molekulargewicht zwischen 63 und 10 000,

c) Inhibitoren bzw. Stabilisatoren gegenüber anionischer Polymerisation sowie

d) weitere Hilfsstoffe wie Stabilisatoren gegen radikalische Polymerisation, Verdickungsmittel, Hilfsmonomere, Farbstoffe und Pigmente.

2. Aerob härtende Kunststoffmassen nach Anspruch 1, dadurch gekennzeichnet, daß sie als Starter Boralkyl- und/oder Borarylverbindungen, Boroxin und/oder Umsetzungsprodukte von Dihydroxyaromaten mit Borwasserstoff enthalten.

3. Aerob härtende Kunststoffmassen nach Anspruch 1, dadurch gekennzeichnet, daß sie durch den bei der Applikation erfolgenden Luftzutritt, vorzugsweise bei Normaltemperatur, härten.

4. Aerob härtende Kunststoffmassen nach Ansprüchen 1 bis 3, dadurch gekennzeichnet, daß sie als polymerisierbare Verbindungen Methacryl- und/oder Acrylsäureverbindungen mit einem Molekulargewicht zwischen 72 und 4 000 und als Starter Boralkylverbindungen enthalten.

5. Aerob härtende Kunststoffmassen nach Ansprüchen 1 bis 4, dadurch gekennzeichnet, daß sie als Methacrylsäure-und/oder Acrylsäureverbindungen Ester mono- und/oder polyfunktioneller Alkohole und/oder gegebenenfalls substituierte Säureamide enthalten.

6. Aerob härtende Kunststoffmassen nach Anspruch 5, dadurch gekennzeichnet, daß ein Anteil der polymerisierbaren (Meth)-acrylsäureverbindungen in Form der freien Säure(n) vorliegt, wobei dieser Anteil vorzugsweise 0,01 bis 10 Gewichtsprozent, insbesondere 0,01 bis 5 Gewichtsprozent beträgt.

7. Aerob härtende Kunststoffmassen nach Ansprüchen 1 bis 6, dadurch gekennzeichnet, daß sie als Verdickungsmittel ein mit den Monomeren homogen mischbares Polymeres, vorzugsweise ein Polymerisat der (Meth)-acrylsäureverbindungen enthalten.

8. Aerob härtende Kunststoffmassen nach Anspruch 7, dadurch gekennzeichnet, daß das Verdickungsmittel ein unter dem Einfluß der Organo-Borverbindungen *in situ* hergestelltes Polymerisat der (Meth)-acrylsäureverbindungen ist.

9. Aerob härtende Kunststoffmassen nach Ansprüchen 1 bis 8, dadurch gekennzeichnet, daß sie die als Starter wirkenden Organo-Borverbindungen in Mengen bis zu 15 Gewichtsprozent, insbesondere von 0,1 bis 10 Gewichtsprozent — bezogen auf Gesamtmenge des polymerisierbaren Anteils — enthalten.

10. Aerob härtende Kunststoffmassen nach Anspruch 1 bis 9, dadurch gekennzeichnet, daß sie eine Viskosität im Bereich von 200 bis 100 000 mPas (vorzugsweise im Bereich von etwa 1 000 bis 20 000 mPas) aufweisen.

11. Verfahren zur Herstellung der fließ- bzw. streichfähigen Kunststoffmassen nach Ansprüchen 1 bis 10, dadurch gekennzeichnet, daß man unter Sauerstoffausschluß einem bei Raumtemperatur flüssigen, vorzugsweise leicht beweglichen Monomeren bzw. Monomeren-Gemisch, das keinen wesentlichen Anteil an freien sauren Gruppen aufweist, mit der bzw. den Organo-Borverbindung(en) versetzt, die Viskosität der Mischung durch Altern in den gewünschten Bereich erhöht, anschließend Inhibitoren gegen anionische Polymerisation zusetzt und das Gemisch gegen radikalische Polymerisation stabilisiert.

12. Verfahren nach Anspruch 11, dadurch gekennzeichnet, daß man die Stabilisierung gegen radikalische Polymerisation durch Zusatz von Borhydrid oder Organo-Borhydridverbindungen vornimmt.

13. Verfahren nach Ansprüchen 11 und 12, dadurch gekennzeichnet, daß man die Borhydridverbindung(en) im Überschuß über die zur Reduktion der vorliegenden Sauerstoffspuren erforderlichen Menge zusetzt.

14. Verfahren nach Ansprüchen 11 bis 13, dadurch gekennzeichnet, daß man als Inhibitoren gegen die anionische Polymerisation Säuren, vorzugsweise eine copolymerisierbare olefinisch ungesättigte Carbonsäure einsetzt.

15. Verwendung der fließ- bzw. streichfähigen Kunststoffmassen nach Ansprüchen 1 bis 10 als aerob aushärtende einkomponentige Reaktionsklebstoffe zum Verbinden von Metall, Holz, Glas, Keramik und Kunststoffen bzw. als chirurgisches Binde- bzw. Klebmittel zum Verbinden von hartem Gewebe, insbesondere Knochen oder als dentalmedizinisches Binde- und Füllmaterial.

## Claims

1. Free-flowing or spreadable synthetic compositions (adhesives, cast resins, fillers) storable in the absence of oxygen and based on polymerizable olefinically unsaturated compounds and organoboron compounds as starters, characterized in that they are in the form of aerobically hardening one-component mixtures essentially consisting of

a) organoboron compounds suitable for initiating the polymerization of ethylenically unsaturated compounds,

b) compounds containing at least one ethylenic bond and having a molecular weight in the range from 63 to 10,000,

c) anionic-polymerization inhibitors or stabilizers and

d) other auxiliaries, such as radical-polymerization stabilizers, thickeners, auxiliary monomers, dyes and pigments.

2. Aerobically hardening synthetic compositions as claimed in Claim 1, characterized in that they contain as starters boron alkyl and/or boron aryl compounds, boroxine and/or reaction products of aromatic dihydroxy compounds with boron hydride.

3. Aerobically hardening synthetic compositions as claimed in Claim 1, characterized in that they harden on contact with air, preferably at room temperature, during application.

4. Aerobically hardening synthetic compositions as claimed in Claims 1 to 3, characterized in that they contain methacrylic and/or acrylic acid compounds having a molecular weight of from 72 to 4 000 as polymerizable compounds and boron alkyl compounds as starters.

5. Aerobically hardening synthetic compositions as claimed in Claims 1 to 4, characterized in that they contain esters of mono- and/or polyfunctional alcohols and/or optionally substituted acid amides as methacrylic acid and/or acrylic acid compounds.

6. Aerobically hardening synthetic compositions as claimed in Claim 5, characterized in that a proportion of the polymerizable (meth) acrylic acid compounds is present in the form of the free acid(s), the proportion in question preferably amounting to between 0.01 and 10 % by weight and more particularly to between 0.01 and 5 % by weight.

7. Aerobically hardening synthetic compositions as claimed in Claims 1 to 6, characterized in that they contain as thickener a polymer homogeneously miscible with the monomers, preferably a polymer of the (meth)acrylic acid compounds.

8. Aerobically hardening synthetic compositions as claimed in Claim 7, characterized in that the thickener is a polymer of the (meth)acrylic acid compounds prepared *in situ* in the presence of the organoboron compounds.

9. Aerobically hardening synthetic compositions as claimed in Claims 1 to 8, characterized in that they contain the organoboron compounds acting as starters in quantities of up to 15 % by weight and more particularly in quantities of from 0.1 to 10 % by weight, based on the total amount of polymerizable material.

10. Aerobically hardening synthetic compositions as claimed in Claims 1 to 9, characterized in that they have a viscosity in the range from 200 to 100,000 mPas and preferably in the range from 1,000 to 20,000 mPas.

11. A process for producing the free-flowing or spreadable synthetic compositions claimed in Claims 1 to 10, characterized in that the organoboron compound(s) is/are added in the absence of oxygen to a monomer or monomer mixture which is liquid and preferably easy to move at room temperature and which does not contain a significant number of free acid groups, the viscosity of the mixture is increased to the required level by ageing, anionic-polymerization inhibitors are subsequently added and the mixture is stabilized against radical polymerization.

12. A process as claimed in Claim 11, characterized in that the mixture is stabilized against radical polymerization by the addition of boron hydride or organic boron hydride compounds.

13. A process as claimed in Claims 11 and 12, characterized in that the boron hydride compound(s) is/are added in an excess over the quantity required to reduce the traces of oxygen present.

14. A process as claimed in Claims 11 to 13, characterized in that an acid, preferably a copolymerizable olefinically unsaturated carboxylic acid, is used as the anionic-polymerization inhibitor.

15. The use of the free-flowing or spreadable synthetic compositions claimed in Claims 1 to 10 as aerobically hardening one-component reactive adhesives for bonding metals, wood, glass, ceramics and plastics or as a surgical adhesive for bonding hard tissue, particularly bones, or as a dental adhesive and filler.

## Revendications

1. Masses de résines synthétiques (colles, résines à couler, matières de charge) fluides ou pouvant être étalées, stables à la conservation à l'abri de l'oxygène, à base de composés à insaturation oléfinique polymérisables et de composés d'organo-bore servant d'inducteurs, caractérisées en ce qu'elles sont sous la forme de mélanges à un composant durcissant à l'air, consistant essentiellement en

a) des composés d'organo-bore convenant pour déclencher une polymérisation de composés à insaturation éthylénique,

b) des composés contenant au moins une liaison éthylénique et ayant un poids moléculaire de 63 à 10 000,

c) des inhibiteurs ou stabilisants contre la polymérisation anionique et

d) d'autres produits auxiliaires tels que des stabilisants contre la polymérisation radicalaire, des agents épaississants, des monomères auxiliaires, des colorants et pigments.

2. Masses de résines synthétiques durcissant à l'air selon la revendication 1, caractérisées en ce qu'elles contiennent en tant qu'inducteurs des composés d'alkyl-bore et/ou d'aryl-bore, une boroxine et/ou des produits de réaction de composés dihydroxyaromatiques avec un borure d'hydrogène.

3. Masses de résines synthétiques durcissant à l'air selon la revendication 1, caractérisées en ce qu'elles durcissent par apport d'air à l'application, de préférence à température normale.

4. Masses de résines synthétiques durcissant à l'air selon les revendications 1 à 3, caractérisées en ce qu'elles contiennent en tant que composés polymérisables des dérivés de l'acide méthacrylique et/ou de l'acide acrylique ayant un poids moléculaire de 72 à 4 000 et en tant qu'inducteurs des composés d'alkyl-bore.

5. Masses de résines synthétiques durcissant à l'air selon les revendications 1 à 4, caractérisées en ce qu'elles contiennent en tant que dérivés de l'acide méthacrylique et/ou de l'acide acrylique des esters d'alcools mono- et/ou poly-fonctionnels et/ou des amides éventuellement substitués.

**0 051 796**

6. Masses de résines synthétiques durcissant à l'air selon la revendication 5, caractérisées en ce qu'une partie des dérivés polymérisables d'acide (méth)acrylique est à l'état du ou des acides libres, cette partie étant de préférence de 0,01 à 10 % en poids, plus spécialement de 0,01 à 5 % en poids.

7. Masses de résines synthétiques durcissant à l'air selon les revendications 1 à 6, caractérisées en ce qu'elles contiennent en tant qu'agent épaississant un polymère donnant des mélanges homogènes avec les monomères, de préférence un polymère de composés (méth)acryliques.

8. Masses de résines synthétiques durcissant à l'air selon la revendication 7, caractérisées en ce qu'elles contiennent en tant qu'agent épaississant un polymère de dérivés de l'acide (méth)-acrylique préparé *in situ* sous l'influence des composés d'organobore.

9. Masses de résines synthétiques durcissant à l'air selon les revendications 1 à 8, caractérisée en ce qu'elles contiennent les composés d'organo-bore faisant fonction d'inducteurs en quantité allant jusqu'à 15 % en poids, plus spécialement de 0,1 à 10 % en poids, par rapport à la quantité totale de la fraction polymérisable.

10. Masses de résines synthétiques durcissant à l'air selon les revendications 1 à 9, caractérisées en ce qu'elles ont une viscosité dans l'intervalle de 200 à 100 000 mPas (de préférence dans l'intervalle d'environ 1 000 à 20 000 mPas).

11. Procédé de préparation des masses de résines synthétiques fluides ou pouvant être étalées selon les revendications 1 à 10, caractérisé en ce que, à un monomère ou mélange de monomères fluide à température ambiante, de préférence bien mobile, ne contenant pas de proportions importantes de groupes acides libres, on ajoute à l'abri de l'oxygène le ou les composés d'organo-bore, on porte la viscosité du mélange par vieillissement dans l'intervalle voulu, on ajoute ensuite les inhibiteurs de polymérisation anionique et on stabilise le mélange contre la polymérisation radicalaire.

12. Procédé selon la revendication 11, caractérisé en ce que la stabilisation contre la polymérisation radicalaire est réalisée par addition d'hydrure de bore ou d'hydrure d'organo-bore.

13. Procédé selon les revendications 11 et 12, caractérisé en ce que le ou les hydrures de bore sont ajoutés en excès par rapport à la quantité nécessaire pour réduire les traces d'oxygène présentes.

14. Procédé selon les revendications 11 à 13, caractérisé en ce que l'on utilise, en tant qu'inhibiteurs de la polymérisation anionique, des acides, et de préférence un acide carboxylique à insaturation oléfinique copolymérisable.

15. Utilisation des masses de résines synthétiques fluides ou pouvant être étalées selon les revendications 1 à 10 en tant que colles réactives à un composant, durcissant à l'air, pour la liaison du métal, du bois, du verre, de la matière céramique et de matières plastiques ou en tant que pansements ou colles chirurgicales pour la liaison des tissus durs, en particulier des os, ou en tant que liants et ciments en médecine dentaire.

11